# FASCICULE DE BREVET EUROPEEN

(11) **EP 0 542 613 B1**
(45) Date de publication et mention de la délivrance du brevet: **28.06.1995**
(21) Numéro de dépôt: 92403021.6
(22) Date de dépôt: 09.11.1992
(51) Int. Cl.: B01J 29/04, B01J 29/40, C07C 2/00

(54) **Catalyseur à structure MFI et son utilisation en aromatisation d'hydrocarbures comportant 2 à 12 atomes de carbone**
Katalysator mit MFI-Struktur und seine Verwendung zum Aromatisieren von Kohlenwasserstoffen mit 2 bis 12 Kohlenstoffatomen
Catalyst with MFI structure and its use for aromatizing hydrocarbons containing 2 to 12 carbon atoms

(30) Priorité: 15.11.1991 FR 9114206; 15.11.1991 FR 9114208
(43) Date de publication de la demande: 19.05.1993
(73) Titulaire: INSTITUT FRANCAIS DU PETROLE, 92502 Rueil-Malmaison (FR)
(72) Inventeur: Alario, Fabio, F-94210 La Varenne (FR); Deves, Jean-Marie, F-78400 Chatou (FR)

(56) Documents cités:
- EP-A- 0 018 498
- EP-A- 0 244 162
- EP-A- 0 458 674
- EP-A- 0 469 951
- US-A- 4 806 701

## Description

La présente invention concerne :
· un catalyseur, dit catalyseur composite, qui contient :
   - une zéolithe de structure MFI contenant au moins un élément choisi dans le groupe constitué par les métaux alcalins et les métaux alcalino-terreux - et dénommé ci-après "alcalin additionnel" -, et contenant dans sa charpente le silicium et au moins un élément choisi dans le groupe formé par l'aluminium et le gallium, ladite zéolithe étant dénommée ci-après "A/MFI",
   - une matrice, laquelle renferme :
   - au moins un métal noble de la famille du platine
   - au moins un métal additionnel choisi dans le groupe constitué par l'étain, le germanium, et le plomb.
   - au moins un halogène choisi dans le groupe constitué par le fluor, le chlore, le brome et l'iode.
   - et éventuellement au moins un élément choisi dans le groupe constitué par les métaux alcalins et les alcalino-terreux.
· sa préparation et son utilisation dans les réactions d'aromatisation des hydrocarbures comportant de 2 à 12 atomes de carbone par molécule et plus particulièrement de 5 à 12 atomes de carbone par molécule, ou 2 à 4 atomes de carbone par molécule selon les types de charges utilisées.

Les métaux alcalins et alcalino-terreux, autres que ceux contenus dans la zéolithe - ces derniers étant appelés alcalin(s) additionnel(s) - ainsi que les métaux de la famille du platine et les métaux additionnels nommés ci-avant, et à l'exception du gallium et du zinc (utilisés à titre d'élément dopeur), sont désignés dans la suite par le terme "métaux".

Les catalyseurs à base de zéolithes dopées au gallium, ou au zinc, ou au platine, sont connus pour être actifs et sélectifs en aromatisation du propane et du butane. Classiquement, les hydrocarbures à plus de 6 atomes de carbone par molécule sont transformés en aromatiques par reformage catalytique en utilisant des catalyseurs du type alumine acide contenant du platine, métal auquel on peut ajouter par exemple de l'étain ou du rhénium. Ces catalyseurs de reformage sont néanmoins très peu performants pour l'aromatisation des hydrocarbures contenant moins de 6 atomes de carbone par molécule. Il y a donc un grand intérêt pratique à trouver des catalyseurs performants pour l'aromatisation des coupes riches en hydrocarbures du type C5-C12.

La réaction d'aromatisation des hydrocarbures contenant moins de 9 atomes de carbone par molécule en présence de zéolithes a déjà fait l'objet de brevets et de publications. Plusieurs systèmes catalytiques à base de zéolithe MFI sont revendiqués, ces systèmes pouvant se distinguer par les ajouts qu'ils contiennent. Schématiquement, on peut distinguer :
(i) les systèmes dopés au gallium (US-A-4175057), et
(ii) les systèmes dopés au zinc (US-A-4288645).

Ces systèmes souffrent tous d'un défaut important, à savoir une sélectivité élevée en méthane. Pour améliorer les performances de ces systèmes catalytiques plusieurs solutions ont été proposées dont l'ajout de platine (Z. Jin, Y. Makino, A. Miyamoto, T. Inui, Chem. Express, 2, p.515, 1987).

Récemment (EP-A-0530069 de la demanderesse qui appartient à l'état de la Technique visé à l'article 54(3) CBE), il a été découvert que l'utilisation de catalyseurs composites contenant une zéolithe MFI d'une part, et d'autre part un support ou une matrice généralement amorphe sur laquelle est déposé un métal noble de la famille du platine et au moins un métal additionnel comme l'étain, le plomb ou l'indium, conduit à des performances catalytiques dans les réactions d'aromatisation des paraffines de 5 à 9 atomes de carbone nettement améliorées par rapport aux systèmes de l'art antérieur.

L'utilisation de tels catalyseurs permet en particulier de limiter les réactions qui conduisent à la formation de méthane, produit non désiré.

Les travaux de recherche effectuées par la demanderesse l'ont conduite à découvrir que, de façon surprenante, l'utilisation d'un catalyseur composite contenant une zéolithe MFI qui contient au moins un élément choisi dans le groupe constitué par les métaux alcalins et les alcalino-terreux, appelée ainsi "A/MFI", éventuellement dopé par du gallium et/ou du zinc sous forme oxyde et une matrice sur laquelle est déposé au moins un métal noble de la famille du platine (notamment palladium, platine, nickel, iridium, rhodium), au moins un métal additionnel choisi dans le groupe constitué par l'étain, le germanium, et le plomb, ladite matrice contenant aussi au moins un halogène (de préférence le chlore) et éventuellement au moins un métal alcalin ou un alcalino-terreux (de préférence le lithium ou le potassium), conduit à des performances catalytiques dans les réactions d'aromatisation des paraffines contenant de 5 à 12 atomes de carbone par molécule nettement améliorées par rapport aux catalyseurs de l'art antérieur.

La zéolithe MFI contenue dans le catalyseur de la présente invention peut être préparée par toutes les techniques décrites dans l'art antérieur. Ainsi la synthèse de ladite zéolithe peut être réalisée en milieu classique OH- en présence ou en absence d'agent organique et/ou d'alcool. Le document "Synthesis of high silica zeolites, P. Jacobs and J. Martens, Studies in Surface Science and Catalysis, Vol. 33, 1987" décrit la synthèse classique de la zéolithe MFI. La zéolithe MFI utilisée dans la présente invention peut également avoir été synthétisée dans des milieux moins classiques comme par exemple le milieu fluorure en présence (brevet EP-A-172068) ou en absence (EP-A-0530069) de composé organique. La zéolithe utilisée dans la présente invention contient, dans sa charpente cristallisée, du silicium et au moins un élément choisi dans le groupe formé par l'aluminium et le gallium.

Après l'étape de synthèse, la zéolithe MFI est :
- soit transformée en une forme hydrogène, notée H-MFI, par élimination pratiquement totale des composés organiques et/ou des cations alcalins ou alcalino-terreux qu'elle contient éventuellement après synthèse. Toutes les techniques décrites dans l'art antérieur peuvent être utilisées pour le passage à la forme hydrogène, comme par exemple les échanges ioniques suivis ou non de calcination ou les traitements chimiques divers. Un ou plusieurs alcalins additionnels sont ensuite introduits par l'intermédiaire de sels en solutions aqueuses ou organiques par toutes les techniques connues de l'art antérieur, pour ensuite obtenir la A/MFI.
- soit directement transformée en une forme contenant un ou plusieurs alcalins additionnels, par élimination totale éventuelle des composés organiques, ce ou ces alcalins additionnels étant alors apportés par le milieu de synthèse de la zéolithe. La teneur en alcalin(s) additionnel(s) peut être alors éventuellement augmentée aisément à partir de sels de ces alcalins ou alcalino-terreux en solutions aqueuses ou organiques par toutes les techniques connues de l'art antérieur, pour ensuite obtenir la A/MFI.

La teneur en alcalin(s) additionnel(s) déposés sur la zéolithe MFI est comprise entre 0,001 et 5 % en poids, de préférence entre 0,005 et 3 % en poids.

Toutes les zéolithes synthétisées dans l'un des systèmes suivants : Si-Al, Si-Al-Ga, Si-Ga, conviennent pour la présente invention. Cependant, leur rapport Si/T où T représente Al et/ou Ga, est généralement supérieur à 7, de préférence supérieur à 10 et de manière encore plus préférée compris entre 13 et 500.

La zéolithe A/MFI, contenant au moins un métal alcalin ou un métal alcalino-terreux, utilisée dans la présente invention, peut être soit éventuellement soumise telle quelle à un dépôt de gallium et/ou de zinc, soit mélangée avec les autres constituants du catalyseur, le gallium et/ou le zinc pouvant alors éventuellement être introduits ultérieurement dans ledit mélange.

Dans le cas où la zéolithe MFI après synthèse est d'abord transformée en sa forme hydrogène, le dépôt éventuel de gallium et/ou de zinc peut intervenir préalablement à l'introduction d'au moins un des métaux alcalins additionnels.

De nombreuses techniques de dépôt de gallium et/ou de zinc peuvent être utilisées dans la présente invention, parmi lesquelles on peut citer les échanges ioniques grâce à l'utilisation de sels en solution aqueuse, ou les imprégnations par des solutions desdits sels.

La teneur cumulée en ces deux métaux éventuellement déposés sur le catalyseur composite est comprise entre 0,01 et 10 % en poids, de préférence entre 0,03 et 4 % en poids.

La matrice comprend au moins un oxyde réfractaire, et en particulier au moins un oxyde d'un métal choisi dans le groupe constitué par le magnésium, l'aluminium, le titane, le zirconium, le thorium, le silicium et le bore. De plus, elle peut aussi comprendre du charbon.

La matrice préférée est l'alumine, dont la surface spécifique peut être avantageusement comprise entre 10 et 600 m2/g et de préférence entre 150 et 400 m2/g.

Le catalyseur composite de la présente invention peut être préparé suivant le procédé dont le principe est donné ci-après, la réalisation pratique étant connue de l'homme de l'art.

On dépose préalablement l'halogène, les métaux et éventuellement l'élément alcalin ou alcalino-terreux sur la matrice d'une part, et éventuellement le gallium et/ou le zinc sur la zéolithe A/MFI d'autre part, ou éventuellement le gallium et/ou le zinc sur la MFI préalablement à l'introduction d'au moins un alcalin additionnel. L'ordre d'introduction des différents éléments sur la matrice est indifférent. Puis on procède au mélange de la zéolithe A/MFI contenant éventuellement du gallium et/ou du zinc avec la matrice contenant les métaux et l'halogène, et à leur mise en forme, la mise en forme étant obtenue dans les mêmes conditions que précédemment.

La méthode préférée de préparation consiste, éventuellement à déposer le gallium et/ou le zinc sur la zéolithe A/MFI, à déposer les métaux et à introduire l'halogène sur la matrice, puis ensuite à introduire la zéolithe éventuellement chargée en gallium et/ou en zinc dans la matrice chargée en métaux et en halogène par mise en forme des deux poudres. La mise en forme est de préférence effectuée après un broyage micronique, qui peut être réalisé en utilisant la technique du broyage humide.

Le catalyseur composite contient entre 1 et 99 % (en poids par rapport à la masse totale du catalyseur) de zéolithe A/MFI le complément à 100 % étant constitué par la matrice, les métaux et l'halogène. La proportion respective de zéolithe et de matrice varie dans une large gamme, car elle dépend d'une part du rapport Si/T de la zéolithe, où T est Al et/ou Ga, et d'autre part de la teneur en métaux et en halogène de la matrice dans le cas de la méthode préférée de préparation.

La matrice contenant les métaux et l'halogène, dans le cas de la méthode préférée de préparation, est généralement préparée suivant les procédures décrites dans EP-A-0530069, dont une partie est reproduite dans ce qui suit. Ces procédures sont utilisables par exemple sur la matrice seule, ou sur le mélange matrice/zéolithe, tel que cela a été décrit dans les deux voies de préparation.

On utilise pour l'imprégnation des métaux, soit une solution commune des métaux que l'on désire introduire, soit des solutions distinctes pour le métal noble de la famille du platine et pour le métal additionnel et éventuellement l'élément choisi dans le groupe constitué par les métaux alcalins et les alcalino-terreux. Quand on utilise plusieurs solutions, on peut procéder à des séchages et/ou à des calcinations intermédiaires. On termine habituellement par une calcination, par exemple entre 500 et 1000 °C, de préférence en présence d'oxygène moléculaire, par exemple en effectuant un balayage d'air.

Le métal noble de la famille du platine peut être incorporé dans la matrice par imprégnation de ladite matrice à l'aide d'une solution, aqueuse ou non, contenant un sel ou un composé du métal noble. Le platine est généralement introduit dans la matrice sous forme d'acide chloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium, le nitrate de palladium.

Le métal additionnel choisi dans le groupe constitué par l'étain, le germanium et le plomb, peut être introduit par l'intermédiaire de composés tels que par exemple les chlorures, les bromures et les nitrates d'étain, les halogénures, le nitrate, l'acétate et le carbonate de plomb, le chlorure et l'oxalate de germanium.

L'halogène peut être introduit à partir d'au moins un des halogénures des métaux de la famille du platine ou d'au moins un des halogénures des métaux additionnels, dans le cas où ces métaux sont introduits à partir d'halogénures. Une méthode complémentaire peut consister en une imprégnation de la matrice par une solution aqueuse contenant un acide ou un sel halogéné. Par exemple, le chlore peut être déposé en utilisant une solution d'acide chlorhydrique. Une autre méthode peut consister en une calcination à une température généralement comprise entre 400 et 900 °C, en présence d'un composé organique contenant l'halogène, comme par exemple CCl₄, CH₂Cl₂, CH₃Cl,...).

L'élément choisi dans le groupe constitué par les alcalins et les alcalino-terreux, de préférence le lithium ou le potassium, peut être introduit par l'intermédiaire de composés tels que par exemple l'halogénure, le nitrate, le carbonate, le cyanure et l'oxalate dudit élément.

Un procédé de préparation, que nous détaillons ci-après, comprend par exemple les étapes suivantes :
a) introduction sur la matrice d'au moins un élément choisi dans le groupe constitué par le fluor, le chlore, le brome et l'iode,
a') Introduction éventuelle sur la matrice d'au moins un élément choisi dans le groupe constitué par les alcalins et les alcalino-terreux.
b) Calcination du produit obtenu à l'étape a) ou a').
c) Introduction sur la matrice d'au moins un métal noble de la famille du platine.
d) Calcination du produit obtenu à l'étape c).
e) Introduction sur le produit obtenu à l'étape d) d'au moins un métal additionnel appelé ci-dessus M.

Si on n'utilise pas de métal alcalin ou alcalino-terreux, on effectue uniquement les étapes a), c), d) et e) dudit procédé de préparation.

L'emploi dans la présente invention d'au moins un métal noble de la famille du platine peut à titre d'exemple se faire grâce à l'utilisation de composés ammoniaqués.

Dans le cas du platine, on peut citer par exemple les sels de platine IV hexamines de formule Pt(NH₃)₆X₄; les sels de platine IV halogénopentamines de formule (PtX(NH₃)₅)X₃: les sels de platine N tétrahalogénodiamines de formule PtX₄(NH₃)₂ ; les complexes de platine avec les halogènes-polycétones et les composés halogénés de formule H(Pt(aca)₂X) ; X étant un halogène choisi dans le groupe formé par le chlore, le fluor, le brome et l'iode , et de préférence X étant le chlore, et aca représentant le reste de formule C₅H₇O₂ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Après introduction du métal noble de la famille du platine, le produit obtenu est éventuellement séché puis il est calciné de préférence à une température comprise entre 400 et 1000 °C.

Après cette calcination, on procède à l'introduction d'au moins un métal additionnel, précédée éventuellement d'une réduction à l'hydrogène à haute température, par exemple entre 300 et 500 °C. Le métal additionnel M peut être introduit sous la forme d'au moins un composé organique choisi dans le groupe constitué par les complexes dudit métal, en particulier les complexes polycétoniques du métal M et les hydrocarbylmétaux tels que les alkyles, les cycloalkyles, les aryles, les alkylaryles et les arylalkyles métaux.

L'introduction du métal M est avantageusement effectuée à l'aide d'une solution du composé organométallique dudit métal dans un solvant organique. On peut également employer des composés organohalogénés du métal M. Comme composés du métal M, on peut citer en particulier le tétrabutylétain dans le cas où M est l'étain, le tétraéthylplomb dans le cas où M est le plomb.

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Dans le cas où l'on n'utilise pas le procédé de préparation tel que décrit ci-dessus, on peut aussi envisager d'introduire au moins un métal additionnel M avant l'introduction d'au moins un métal noble de la famille de platine. Si le métal M est introduit avant le métal noble, le composé du métal M utilisé est généralement choisi dans le groupe constitué par l'halogénure, le nitrate, l'acétate, le tartrate, le carbonate et l'oxalate du métal M. L'introduction est alors avantageusement effectuée en solution aqueuse. Dans ce cas, avant de procéder à l'introduction d'au moins un métal noble, on procède à une calcination sous air à une température comprise entre 400 et 1000 °C.

Le catalyseur composite à base d'une MFI et d'une matrice contient de préférence :
1) en poids par rapport à la matrice:
   - de 0,01 à 2 % et de préférence de 0,1 à 0,5 % d'au moins un métal noble de la famille du platine, déposé dans la matrice et/ou la zéolithe,
   - déposé dans la matrice et/ou la zéolithe, au moins un métal additionnel à savoir de 0,005 à 2 %, de préférence de 0,01 à 0,5 %, d'étain dans le cas où le métal additionnel est de l'étain, de 0,005 à 0,7 %, de préférence de 0,01 à 0,6 %, d'au moins un métal choisi dans le groupe constitué par le germanium et le plomb; dans le cas où le catalyseur contient au moins deux métaux additionnels dudit groupe, la teneur globale en métaux additionnels choisis est comprise entre 0,02 et 1,20 %, de préférence entre 0,02 et 1,0 %, et de manière encore plus préférée entre 0,03 et 0,80 %.
   - de 0,1 à 15 %, et de préférence de 0,2 à 10 %, d'un élément halogène choisi dans le groupe constitué par le fluor, le chlore, le brome et l'iode, de préférence le chlore, l'halogène étant incorporé dans la matrice et/ou la MFI.
   - éventuellement de 0,01 à 4 %, de préférence de 0,1 à 0,6 % d'au moins un métal choisi dans le groupe constitué par les alcalins et les alcalino-terreux, de préférence choisi dans le groupe constitué par le lithium et le potassium.
2) entre 1 et 99% en poids de zéolithe MFI, contenant dans sa charpente du silicium et au moins un élément choisi parmi l'aluminium et le gallium,
3) éventuellement en poids par rapport à la zéolithe :
   - entre 0,001 et 5%, et de préférence entre 0,005 et 3% d'au moins un alcalin additionnel choisi dans le groupe constitué par les alcalins et alcalino-terreux,
   - éventuellement encore entre 0,01 et 10% en poids, de préférence entre 0,03 et 4%, d'un élément dopeur choisi dans le groupe constitué par le gallium et le zinc, de préférence le gallium, cet élément étant introduit dans la MFI.

A l'issue de la préparation, le catalyseur mis en forme contient une zéolithe MFI, des métaux, au moins un halogène et une matrice, et on procède à une calcination sous air à une température comprise entre 450 et 1000 °C. Le catalyseur ainsi calciné peut avantageusement subir un traitement d'activation sous hydrogène à haute température, par exemple comprise entre 300 et 500 °C. La procédure de traitement sous hydrogène consiste par exemple en une montée lente de la température sous courant d'hydrogène jusqu'à la température maximale de réduction, comprise généralement entre 300 et 500 °C et de préférence entre 350 et 450 °C, suivie d'un maintien à cette température pour une durée comprise en général entre 1 et 6 heures.

Le catalyseur de la présente invention décrit précédemment est mis en oeuvre pour l'aromatisation des alcanes contenant 2 à 12, par exemple 2 à 4 ou 5 à 12 atomes de carbone par molécule, en présence ou non d'oléfines. Cette réaction revêt un intérêt particulier car elle peut permettre, par exemple, d'une part de valoriser des fractions légères provenant d'opérations de raffinage en les transformant en des produits à plus haute valeur ajoutée (benzène, toluène et xylènes), d'autre part de transformer des charges paraffiniques pouvant contenir des oléfines en bases pour carburant à haut indice d'octane, tout en contribuant dans les deux cas à la production de quantités importantes d'hydrogène indispensable pour les procédés d'hydrotraitement par exemple.

La charge qui contient des composés contenant de 5 à 12 atomes de carbone par molécule est mise en contact avec le catalyseur de la présente invention à une température comprise entre 400 et 700 °C.

Les exemples qui suivent précisent l'invention sans toutefois en limiter la portée.

### Exemple 1 : préparation de l'alumine renfermant du platine, de l'étain et du chlore (catalyseur A).

L'alumine utilisée a une surface spécifique de 220 m2/g et un volume poreux de 0,52 cm3/g.

A 100 g de support d'alumine on ajoute 500 cm3 d'une solution aqueuse d'acide chlorhydrique. On laisse en contact 3 heures, on essore, on sèche 1 heure à 100-120°C.

Sur le produit séché contenant le chlore, on procède alors à l'imprégnation du platine, en ajoutant au solide 150 cm3 d'une solution aqueuse d'acide hexachloroplatinique. La concentration en platine de cette solution est égale à 2.7 g/l. On laisse 6 heures en contact, on sèche 1 heure à 100-120°C puis on calcine 2 heures à 530°C

Sur le produit calciné contenant le chlore et le platine, on procède à l'imprégnation de l'étain : une solution organique de tétrabutylétain est mise en contact avec le support d'alumine à raison de 100 cm3 de solution pour 100 g de support pendant 6 heures. Le solide obtenu est alors essoré et séché 1 heure à 100-120°C puis réduit sous courant d'hydrogène sec pendant 2h à 450°C.

L'alumine contient alors, en poids, 0,40 % de platine, 0,3 % d'étain et 1,0 % de chlore.

### Exemple 2 : zéolithe MFI forme hydrogène (catalyseur B).

On utilise une zéolithe H-MFI forme hydrogène qui est obtenue de la forme NaMFI issue de la synthèse de la façon suivante :
- traitement au nitrate d'ammonium
- calcination à 550°C sous air.

Les caractéristiques du solide obtenu sont : un rapport Si/Al égal à 29 et une teneur en sodium égale à 0,014 % en poids. Son volume poreux mesuré par adsorption d'azote à 77K est de 0,192 cm3/g. Le volume de maille du réseau cristallin est de 5339 Angstroem cube.

### Exemple 3 : préparation d'une zéolithe MFI forme hydrogène renfermant du sodium (catalyseur C).

Cette zéolithe est préparée à partir d'une zéolithe H-MFI forme hydrogène sur laquelle le sodium est déposé à l'aide d'une solution aqueuse de nitrate de sodium. La concentration de cette solution est égale à 48,5 g/l. Celle ci est mise en contact avec la zéolithe HMFI pendant 6 heures. Le solide est ensuite essoré, séché à température voisine de 120°C puis calciné à 550°C sous air.

Les caractéristiques du solide obtenu sont : un rapport Si/Al égal à 29 et une teneur en sodium égale à 0,25 % en poids. Son volume poreux mesuré par adsorption d'azote à 77K est de 0,189 cm3/g.

### Exemple 4 : préparation des mélanges (catalyseurs E et F)

Préparation de l'alumine renfermant du platine et de l'étain (catalyseur D).

L'alumine utilisée est identique à celle utilisée dans l'exemple 1.

Sur 100g de cette alumine on procède à l'imprégnation du platine, en ajoutant au solide 150 cm3 d'une solution aqueuse d'acide hexachloroplatinique. La concentration en platine de cette solution est égale à 2,7 g/l. On laisse 6 heures en contact, on sèche 1 heure à 100-120°C puis on calcine 2 heures à 530°C

Sur le produit calciné contenant le platine, on procède à l'imprégnation de l'étain : une solution organique de tétrabutylétain est mise en contact avec le support d'alumine à raison de 100 cm3 de solution pour 100 g de support pendant 6 heures. Le solide obtenu est alors essoré et séché 1 heure à 100-120°C puis réduit sous courant d'hydrogène sec pendant 2 heures à 450°C.

L'alumine ainsi préparée (catalyseur D) contient alors, en poids, 0,40 % de platine, et 0,3 % d'étain.

### Préparation des mélanges (catalyseurs E et F)

Les catalyseurs E et F sont préparés par mélange à partir des catalyseurs A, C et D. Ces mélanges sont réalisés dans les proportions massiques suivantes :
- catalyseur E = 60 g catalyseur A + 40 g catalyseur C
- catalyseur F = 60 g catalyseur D + 40 g catalyseur C
Chacun des constituants de ces mélanges est soumis au préalable à un broyage submicronique. Après mélange, les catalyseurs E et F sont mis en forme par pastillage.

### Exemple 5: performances des catalyseurs sur charge à 5 et 6 atomes de carbone par molécule.

On se propose de transformer une charge constituée d'un mélange d'hydrocarbures contenant de 5 à 6 atomes de carbone par molécule. On opère pour cela en présence d'un des catalyseurs A, B, C, E et F dont la préparation a été décrite précédemment.

Ces catalyseurs ont été testés en transformation d'une charge C5-C6 dont la composition est la suivante (exprimée en % poids) :

| | | |
|---|---|---|
| Paraffines | C5 | 90 % |
| | C6 | 5,4% |
| Naphtènes | C5 | 3,7 % |
| | C6 | 0,9% |

Les conditions opératoires sont les suivantes :

| | |
|---|---|
| - température | 460°C |
| - pression | 2,5 bars |
| - pph | 2 h-1 |

Les résultats du test sont reportés dans le tableau 1.

| | | Sélectivités (% poids) | | | | | |
|---|---|---|---|---|---|---|---|
| Catalyseur | Conversion (% poids) | CH₄ | C₂H₆ + C₂H₄ | C₃H₈ + C₃H₆ | butanes + butènes | oléfines C₅ + C₆ | Aromatiques |
| Catalyseur A (comparatif) | 53 | 3 | 11 | 20 | 17 | 39 | 10 |
| Catalyseur B (comparatif) | 89 | 28 | 26 | 15 | 20 | 0 | 11 |
| Catalyseur C (comparatif) | 81 | 25 | 26 | 16 | 21 | 0 | 12 |
| Catalyseur E | 76 | 8 | 16 | 18 | 12 | 1 | 45 |
| Catalyseur F (comparatif) | 72 | 8 | 13 | 19 | 15 | 6 | 39 |

On constate donc que le catalyseur E selon l'invention conduit à des conversions et à des sélectivités en produits aromatiques nettement supérieures à celles des catalyseurs comparatifs, notamment du catalyseur F.

### Exemple 6 : performances des catalyseurs sur charge à 3 atomes de carbone par molécule.

Les cinq catalyseurs A, B, C, E et F dont la préparation a été décrite précédemment, ont été testés en transformation du propane dans les conditions suivantes :

| | |
|---|---|
| - température | 475°C |
| - pression | atmosphérique |
| - pph | 1,0 h-1 |

Les résultats du test sont reportés dans le tableau 2.

**Tableau 2**

| | | Sélectivités (% moles) | | | | |
|---|---|---|---|---|---|---|
| Catalyseur | Conversion (% moles) | méthane | éthane + éthylène | propylène | butanes + butènes | Aromatiques |
| Catalyseur A (comparatif) | 15 | 2 | 6 | 90 | 0 | 2 |
| Catalyseur B (comparatif) | 22 | 48 | 27 | 22 | 1 | 2 |
| Catalyseur C (comparatif) | 20 | 45 | 27 | 23 | 3 | 2 |
| Catalyseur E | 36 | 8 | 26 | 22 | 4 | 40 |
| Catalyseur F (comparatif) | 26 | 8 | 23 | 33 | 2 | 34 |

On constate donc que le catalyseur E selon l'invention conduit à des conversions et à des sélectivités en produits aromatiques nettement supérieures à celles des catalyseurs comparatifs, notamment du catalyseur F.

## Revendications

1. Catalyseur composite renfermant d'une part une zéolithe de structure MFI contenant dans sa charpente du silicium et au moins un élément choisi dans le groupe constitué par l'aluminium et la gallium et d'autre part une matrice, la dite zéolithe renfermant au moins un élément appelé alcalin additionnel choisi dans le groupe constitué par les métaux alcalins et les métaux alcalinoterreux, le catalyseur renfermant en outre :
- au moins un métal noble de la famille du platine, déposé sur la matrice
- au moins un métal dit additionnel déposé sur la matrice, le dit métal étant choisi dans le groupe constitué par l'étain, le germanium et, le plomb.
- au moins un élément halogène déposé sur la matrice choisi dans le groupe constitué par le fluor, le chlore, le brome et l'iode.

2. Catalyseur selon la revendication 1 et renfermant en outre un élément dopeur choisi dans le groupe constitué par le gallium et le zinc, l'élément dopeur étant déposé dans la la zéolithe.

3. Catalyseur composite selon l'une des revendications 1 et 2 dans lequel la teneur en zéolithe représente 1 à 99 % en poids par rapport à la masse totale du catalyseur.

4. Catalyseur selon l'une des revendications 1 à 3 dans lequel la teneur en alcalin additionnel est comprise entre 0,001 et 5 % en poids par rapport la zéolithe.

5. Catalyseur selon l'une des revendications 1 à 4 dans lequel la teneur en métal noble de la famille du platine est comprise entre 0,01 et 2 %, exprimée en poids par rapport à la matrice.

6. Catalyseur selon l'une des revendications 1 à 5 dans lequel la teneur en métal additionnel exprimée en poids par rapport à la matrice est comprise entre :
- 0,005 et 2 % lorsque le métal additionnel est l'étain
- 0,005 à 0,7 % lorsque le métal additionnel est choisi parmi les autres métaux additionnels

7. Catalyseur selon l'une des revendications 1 à 6 dans lequel lorsqu'il y a présence au moins de deux métaux additionnels, la teneur globale en métaux additionnels, en poids par rapport à la matrice, est comprise entre 0,02 et 1,20%.

8. Catalyseur selon l'une des revendications 1 à 7 dans lequel la teneur en halogène (exprimée en poids par rapport à la matrice) est comprise entre 0,1 et 15 %.

9. Catalyseur selon l'une des revendications 1 à 8 dans lequel la teneur en élément dopeur, exprimée en poids par rapport à la zéolithe est comprise entre 0,01 et 10 %.

10. Catalyseur selon l'une des revendications 1 à 9 dans lequel la matrice est une alumine.

11. Utilisation du catalyseur selon l'une des revendications 1 à 10 dans un procédé d'aromatisation d'hydrocarbures comportant de 2 à 12 atomes de carbone par molécule.

## Claims

1. Composite catalyst containing on the one hand a MFI structure zeolite containing in its skeleton silicon and at least one element chosen from within the group constituted by aluminium and gallium and on the other a matrix, said zeolite containing at least one element referred to as the additional alkali and chosen from within the group constituted by alkali metals and alkaline earth metals, said catalyst also containing:
at least one precious metal from the platinum group, deposited on the matrix,
at least one so-called additional metal deposited on the matrix said metal being chosen from within the group constituted by tin, germanium and lead,
at least one halogen element deposited on the matrix and chosen from within the group constituted by fluorine, chlorine, bromine and iodine.

2. Catalyst according to claim 1 and also containing a doping element chosen from within the group constituted by gallium and zinc, the doping element being deposited in the zeolite.

3. Composite catalyst according to either of the claims 1 and 2, wherein the zeolite content represents 1 to 99% by weight, based on the total weight of the catalyst.

4. Catalyst according to any one of the claims 1 to 3, wherein the additional alkali content is between 0.001 and 5% by weight, based on the zeolite.

5. Catalyst according to any one of the claims 1 to 4, wherein the content of the precious metal from the platinum group is between 0.01 and 2% by weight based on the matrix.

6. Catalyst according to any one of the claims 1 to 5, wherein the weight content of the additional metal based on the matrix is between 0.005 and 2% when the additional metal is tin and 0.005 and 0.7% when the additional metal is chosen from among the other additional metals.

7. Catalyst according to any one of the claims 1 to 6, wherein when at least two additional metals are present, the total weight content of additional metals based on the matrix is between 0.02 and 1.20%.

8. Catalyst according to any one of the claims 1 to 7, wherein the halogen content (by weight based on the matrix) is between 0.1 and 15%.

9. Catalyst according to any one of the claims 1 to 8, wherein the doping element content, expressed by weight based on the zeolite, is between 0.01 and 10%.

10. Catalyst according to any one of the claims 1 to 9, wherein the matrix is an alumina.

11. Use of the catalyst according to any one of the claims 1 to 10 in a process for the aromatization of hydrocarbons having 2 to 12 carbon atoms per molecule.

## Patentansprüche

1. Verbundkatalysator, der einerseits einen Zeolith der MFI-Struktur, der in seinem Gitter Silicium und mindestens ein Element enthält, das aus der von Aluminium und Gallium gebildeten Gruppe ausgewählt wird, und andererseits eine Matrix umfaßt, der besagte Zeolith umfaßt mindestens ein als zusätzliches Alkali bezeichnetes Element, das aus der von den Alkali- und Erdalkalimetallen gebildeten Gruppe ausgewählt wird, wobei der Katalysator außerdem umfaßt:
- mindestens ein Edelmetall aus der Gruppe der Platinmetalle, das auf der Matrix abgelagert ist
- mindestens ein sogenanntes zusätzliches Metall, das auf der Matrix abgelagert ist, wobei das besagte Metall aus der von Zinn, Germanium und Blei gebildeten Gruppe ausgewählt wird
- mindestens ein Halogen, das auf der Matrix abgelagert ist und aus der von Fluor, Chlor, Brom und Iod gebildeten Gruppe ausgewählt wird.

2. Katalysator nach Anspruch 1, der außerdem ein Dotierungselement enthält, das aus der von Gallium und Zink gebildeten Gruppe ausgewählt wird, wobei das Dotierungselement auf dem Zeolith abgelagert ist.

3. Verbundkatalysator nach einem der Ansprüche 1 und 2, in dem der Zeolithgehalt 1 bis 99 Gew.-%, bezogen auf die Gesamtmasse des Katalysators, darstellt.

4. Katalysator nach einem der Ansprüche 1 bis 3, bei dem der Gehalt an zusätzlichem Alkali zwischen einschließlich 0.001 und 5 Gew.-%, bezogen auf Zeolith, beträgt.

5. Katalysator nach einem der Ansprüche 1 bis 4, bei dem der Gehalt an Edelmetall aus der Gruppe der Platinmetalle zwischen einschließlich 0.01 und 2 % beträgt, ausgedrückt in Gew.-% bezogen auf die Matrix.

6. Katalysator nach einem der Ansprüche 1 bis 5, bei dem der Gehalt an zusätzlichem Metall, ausgedrückt in Gew.-% bezogen auf die Matrix zwischen einschließlich
- 0.005 und 2 % liegt, wenn das zusätzliche Metall Zinn ist
- 0.005 und 0.7 % liegt, wenn das zusätzliche Metall aus den anderen zusätzlichen Metallen ausgewählt wird.

7. Katalysator nach einem der Ansprüche 1 bis 6, bei dem, wenn mindestens zwei zusätzliche Metalle enthalten sind, der Gesamtgehalt an zusätzlichen Metallen in Gew.-%, bezogen auf die Matrix, zwischen einschließlich 0.02 und 1.20 % liegt.

8. Katalysator nach einem der Ansprüche 1 bis 7, bei dem der Gehalt an Halogen (ausgedrückt in Gew.-% bezogen auf die Matrix) zwischen einschließlich 0.1 und 15 % liegt

9. Katalysator nach einem der Ansprüche 1 bis 8, bei dem der Gehalt an Dotierungselement, ausgedrückt in Gew.-% bezogen auf den Zeolith, zwischen einschließlich 0.01 und 10 % liegt.

10. Katalysator nach einem der Ansprüche 1 bis 9, bei dem die Matrix ein Aluminiumoxid ist.

11. Verwendung des Katalysators nach einem der Ansprüche 1 bis 10 in einem Aromatisierungsverfahren von Kohlenwasserstoffen, die zwischen 2 und 12 Kohlenstoffatomen pro Molekül enthalten.
